Europäisches Patentamt

European Patent Office

Office européen des brevets

(11) Publication number: **0 145 489**
**B1**

(12) **EUROPEAN PATENT SPECIFICATION**

(45) Date of publication of patent specification: **07.06.89**

(21) Application number: **84308636.4**

(22) Date of filing: **12.12.84**

(51) Int. Cl.⁴: **A 61 M 25/00**

(54) **Floppy guide wire with opaque tip.**

(30) Priority: **12.12.83 US 560802**

(43) Date of publication of application:
**19.06.85 Bulletin 85/25**

(45) Publication of the grant of the patent:
**07.06.89 Bulletin 89/23**

(84) Designated Contracting States:
**CH DE FR GB LI**

(56) References cited:
**GB-A-2 127 294**
**US-A-3 452 742**
**US-A-3 547 103**
**US-A-3 749 086**
**US-A-3 841 308**
**US-A-4 080 706**

(73) Proprietor: **ADVANCED CARDIOVASCULAR SYSTEMS, INC.**
**1395 Charleston Road**
**Mountain View, CA 94039-7101 (US)**

(72) Inventor: **Morrison, David W.**
**5509 Willowbrook Drive**
**San Jose, CA 95118 (US)**
Inventor: **Samson, Wilfred J.**
**12715 Fredericksburg Drive**
**Saratoga, CA 95070 (US)**

(74) Representative: **Bizley, Richard Edward et al**
**BOULT, WADE & TENNANT 27 Furnival Street**
**London EC4A 1PQ (GB)**

Courier Press, Leamington Spa, England.

## Description

This invention relates to a guide wire for use in introducing catheters into vascular systems, for example into cardiovascular systems in humans, and comprising:

i) a flexible metal elongate core element having a cylindrical proximal portion and a tapered portion adjoining the distal end of the cylindrical proximal portion;

ii) a helically coiled element which is secured to the core element, extends around and is generally co-axial with the core element and extends beyond the distal end of the core element, which coiled element carries a rounded metal tip at its free distal end; and

iii) a safety wire which is secured to the rounded tip, extends inside the coiled element and is secured to the core element.

Such a guide wire is disclosed in US-A-3547103 (Cook), the tapered portion providing the distal end of the core element. The safety wire extends along the whole length of the core element and is either just secured at the proximal end of the core element or the core element, the safety wire and the helically coiled element are soldered together at intervals along their length.

One object of the invention is to provide a guide wire which has a very flexible or floppy distal end to facilitate travel in the small vessels of vascular systems.

Another object of the invention is to provide a guide wire in which the flexible distal end has a greater flexibility or freedom of movement in one direction than another.

The invention is characterised in that:

iv) the core element has a flattened distal portion adjoining the distal end of the tapered portion; and

v) the safety wire is secured to the core element at a position which is between and spaced from the proximal portion and the flattened distal portion of the core element.

An embodiment of the invention will now be described by way of example and with reference to the accompanying drawings, in which:

Figure 1 is a side elevational view partially in cross section of a guide wire;

Figure 2 is a cross sectional view taken along the line 2-2 of Figure 1; and

Figure 3 is an enlarged view of the distal portion of the guide wire shown in Figure 1.

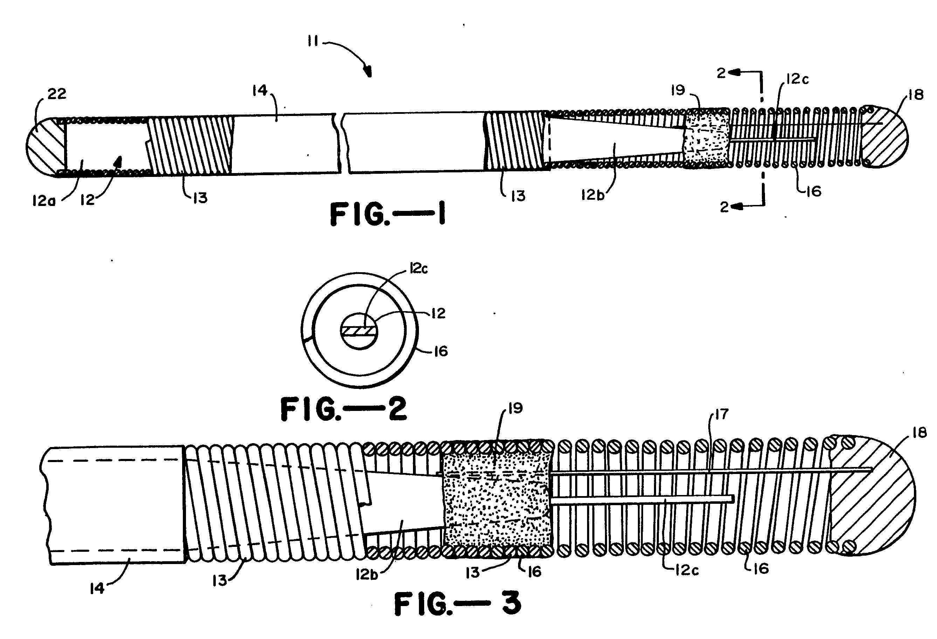

The drawings illustrate a guide wire 11 which comprises a flexible elongate core element 12 formed of a suitable metallic material having a high torsional strength, such as stainless steel. Other materials can be utilised such as certain carbon steels as well as titanium or beryllium copper. The flexible elongate core element 12 can be in the form of a wire-like hollow cylindrical element or in the form of a wire-like solid cylindrical core. One particular material utilised for the flexible elongate element 12 has been No. 304 stainless steel which includes approximately 20% chromium and 10% nickel and has a minimum of 1.655GPa (240 ksi) tensile strength. The material is preferably work-hardened by drawing 3.8mm (0.15 inch) stainless steel wire down to wire having a diameter of 0.25mm (0.01 inches). The wire after being work-hardened is straightened and cut to the desired length. Thereafter, it is then centerless ground and the tip flattened to provide the shape shown in Figure 1.

The guide wire 11 has a suitable length which depends upon the application for which it is used. A typical guide wire can have a length of approximately 175cm ± 5cm. The flexible elongate element 12 has a total length of slightly less than the 175cm as, for example, a length of approximately 173.5cm for a purpose hereinafter described. The flexible elongate core element 12 is provided with an elongate generally cylindrical proximal portion 12a which extends almost the entire length of the guide wire 11. It is adjoined by an intermediate tapered portion 12b which in turn is adjoined by a distal flattened portion 12c. Thus the portions 12b and 12c form the distal portion of the flexible elongate element 12. The cylindrical portion 12a has suitable dimensions such as a diameter ranging from 0.013mm to 0.3mm (0.005 to 0.012 inches) and preferably a diameter of approximately 0.25mm (0.01 inches). The tapered portion 12b tapers down from a larger diameter to a smaller diameter as, for example, down to 0.076 and 0.102mm (0.003 and 0.004 inches). The tapered portion can have a suitable length as, for example, 40mm ± 20mm. The flattened portion 12c can have a suitable length such as 55mm ± 20mm. The flattened portion 12c typically can be formed by centerless grinding to a uniform dimension of 0.076 to 0.102mm (0.003 to 0.004 inches) in diameter, after which this portion is stamped or rolled flat. When so flattened, the portion 12c can have a length of approximately 15mm ± 10mm. It can also have a thickness of approximately 0.05mm (0.002 inches) and a width from 0.05 to 0.2mm (0.002 to 0.008 inches) depending upon the diameter of the starting wire. When the distal extremity 12c of the flexible elongate core element has been flattened in this manner, this distal portion will have much greater flexibility of movement in a direction which is perpendicular to the width of the flattened portion than in other directions and in particular, parallel to the plane of the flattened portion.

An elongate helically coiled element 13, which is formed of a suitable material such as stainless steel, is coaxially disposed around the flexible elongate core element 12 and extends substantially the entire length thereof from the proximal end to near the commencement of the tapered portion 12b. The coil 13 can be formed of wire having a diameter from 0.025 to 0.127 mm (0.001 to 0.005 inches) and preferably a diameter of 0.076 mm (0.003 inches). With such dimensions the coil would have an outer diameter ranging from 0.178mm to 0.64mm (0.007 to 0.025 inches). The coil 13 is tightly wound so that the turns are bottomed out or butt each other. In order to provide a smooth relatively slippery or unctuous surface, a coating 14 of a suitable material such as PTFE is provided on the coil 13 to generally provide for lubricity when exposed to blood and contrast agents which are notably sticky.

Another elongate helically coiled element 16 is provided which adjoins the coil 13. The coil 16 is formed of material which is substantially opaque to x-rays. For that reason it should be formed of a material which has a density of at least 13 grams/$cm^3$. Suitable materials meeting this requirement include gold, tantalum, tungsten, platinum, iridium, rhenium and alloys of these materials. The coil 16 has dimensions substantially the same as the dimensions of the wire utilised for forming the coil 13. The proximal portion of the coil 16 was formed with turns which are bottomed out on each other. However, near the distal end of the coil 16, the turns are stretched or spaced apart a suitable distance as, for example 0.076mm ± 0.0076mm (0.003 inches ± 0.0003 inches). This spacing of the turns of the coil 16 provides additional flexibility in its distal extremity. The distal extremity of the coil 13 and the proximal extremity of the coil 16 are threaded together as shown particularly in Figures 1 and 3 so that a connection is formed therebetween. Alternatively, if desired, the two coils 13 and 16 can have these same ends butted together.

A safety ribbon on wire 17, which is formed of a suitable material such as tungsten, is provided and is disposed internally of the coil 13. It can have suitable dimensions such as a thickness of 0.025 mm (0.001 inches) and a width of 0.076mm (0.003 inches). A rounded metal tip or protrusion 18 in the form of a gold alloy slug is disposed in the distal extremity of the coil 16. Typically, this is formed by brazing the small slug 18 into the distal extremity of the coil. The safety ribbon 17 is brazed into the slug 18 and extends rearwardly from the slug 18 into a region where it overlies the tapered portion 12b of the element 12. The distal extremity of the coil 13, the proximal extremity of the coil 16 and the proximal extremity of the safety ribbon 17 and the tapered portion 12b of the core element 12 are bonded into a unitary assembly by a brazing compound 19.

A rounded metal tip or protrusion 22, which is formed of a suitable material such as gold is secured to the proximal extremity of the coil 13 by suitable means such as brazing.

According to the construction herein described, the guide wire 11 has a substantially continuous diameter throughout its entire length. The transition between the stainless steel coil 13 and the platinum coil 16 can only be denoted by the brazing 19.

The brazing 19 is applied in such a manner that a smooth surface is provided by the outer surface of the brazing so that a substantially smooth, relatively slippery exterior surface is provided by the guide wire 11.

The guide wire 11 hereinbefore described can be utilized as a guide wire for inserting catheters in vascular systems and particularly into cardiovascular systems. The entire guide wire is relatively floppy and can readily be introduced in a manner well known to those skilled in the art. The distal extremity of the guide wire is flexible. The flexibility is enhanced because the distal extremity of the coil 16 is spaced from the flattened portion 12c of the flexible element 12. The tungsten safety ribbon 17, in addition to providing a safety ribbon which prevents extension of the coil 13 when it is being retracted and from becoming separated from the flexible elongate element 12, is pliable and can be shaped by hand so as to provide the distal extremity of the coil 16 with a pre-determined shape. Thus in addition to being very flexible, the distal extremity of the guide wire 11 can be pre-shaped. The flattened portion 12c of the element 12 provides extreme flexibility in one direction, namely in a direction perpendicular to the plane of the flattened portion 12c while at the same time providing some rigidity in other directions to facilitate negotiation of difficult regions in the vascular system. Since the distal extremity of the guide wire 11 is relatively light, it floats in a moving blood stream as, for example, one encountered in the human cardiovascular system which facilitates movement of the guide wire through the vessels of the vascular system.

The construction of the guide wire is relatively simple and is one which can be repeatably manufactured with consistency.

In summary, the guide wire is floatable in the blood stream. The tip of the guide wire can be pre-shaped by the doctor or radiologist so as to be able to enter different areas of the cardiovascular system. The guide wire is still stiff enough so it can be used as a guide and still floppy enough so that it will not penetrate the wall of the vessel. The guide wire is sufficiently small so that it can penetrate lesions and still permit blood flow through an occluded vessel. The tip of the guide wire is substantially radiopaque so that its travel can be observed under conventional fluoroscopy. By way of example, in the event that the guide wire reaches a dead end, a shepherd's hook will be formed in the end of the guide wire which can be observed on the fluoroscope. Because of the ability of the distal extremity of the guide wire to float, it can find and pass through eccentric openings in the vessels.

**Claims**

1. A guide wire comprising:

i) a flexible metal elongate core element (12) having a cylindrical proximal portion (12a) and a tapered portion (12b) adjoining the distal end of the cylindrical proximal portion;

ii) a helically coiled element (16) which is secured to the core element (12), extends around and is generally co-axial with the core element (12) and extends beyond the distal end of the core element (12), which coiled element (16) carries a rounded metal tip (18) at its free distal end; and

iii) a safety wire (17) which is secured to the rounded tip (18), extends inside the coiled element (16) and is secured to the core element (12);

characterised in that:

iv) the core element (12) has a flattened distal portion (12c) adjoining the distal end of the tapered portion (12b); and

v) the safety wire (17) is secured to the core element (12) at a position which is between and spaced from the proximal portion (12a) and the flattened distal portion (12c) of the core element.

2. A guide wire as claimed in claim 1, characterised in that the safety wire (17) is secured to the tapered portion (12b) of the core element (12).

3. A guide wire as claimed in claim 1 or 2, characterised in that a second, metal helically coiled element (13) is secured to the core element (12), extends around the proximal portion (12a) and is generally co-axial with the core element (12) and adjoins the first coiled element (16).

4. A guide wire as claimed in claim 3, characterised in that a brazing compound (19) bonds together the adjoining ends of the first and second coiled elements (16, 13).

5. A guide wire as claimed in any preceding claim, characterised in that the turns of the first coiled element (16) near the distal end are spaced further apart than the turns near the proximal end.

6. A guide wire as claimed in any preceding claim, characterised in that the safety wire (17) is formed of a pliable metal enabling the distal end of the device to be pre-shaped.

**Patentansprüche**

1. Führungsdraht mit

i) einem flexiblen langgestreckten Metallkernelement (12), das einen zylindrischen proximalen Teil (12a) und einen konischen Teil (12b) neben dem distalen Ende des zylindrischen proximalen Teils aufweist,

ii) einem schraubenförmig gewickelten Element (16), das am Kernelement (12) befestigt ist, um das Kernelement (12) und im wesentlichen koaxial dazu verläuft und sich über das distale Ende des Kernelementes (12) hinaus erstreckt, welches gewickelte Element (16) eine abgerundete Metallspitze (18) an seinem freien distalen Ende trägt, und

iii) einem Sicherheitsdraht (17), der an der abgerundeten Spitze (18) befestigt ist, im Inneren des gewickelten Elementes (16) verläuft und am Kernelement (12) befestigt ist,

dadurch gekennzeichnet, daß

iv) das Kernelement (12) einen abgeflachten distalen Teil (12c) neben dem distalen Ende des konischen Teils (12b) aufweist und

v) der Sicherheitsdraht (17) am Kernelement (12) an einer Stelle befestigt ist, die zwischen dem proximalen Teil (12a) und dem abgeflachten distalen Teil (12c) des Kernelementes und im Abstand davon liegt.

2. Führungsdraht nach Anspruch 1, dadurch gekennzeichnet, daß der Sicherheitsdraht (17) am konischen Teil (12b) des Kernelementes (12) befestigt ist.

3. Führungsdraht nach Anspruch 1 oder 2, dadurch gekennzeichnet, daß ein zweites schraubenförmig gewickeltes Metallelement (13) am Kernelement (12) befestigt ist, um den proximalen Teil (12a) herum und im wesentlichen koaxial zum Kernelement (12) verläuft und sich an das erste gewickelte Element (16) anschließt.

4. Führungsdraht nach Anspruch 3, dadurch gekennzeichnet, daß eine Lötmasse (19) die benachbarten Enden des ersten und zweiten gewickelten Elementes (16, 13) miteinander verbindet.

5. Führungsdraht nach einem der vorhergehenden Ansprüche, dadurch gekennzeichnet, daß die Windungen des ersten gewickelten Elementes (16) nahe am distalen Ende einen größeren Abstand als die Windungen nahe am proximalen Ende haben.

6. Führungsdraht nach einem der vorhergehenden Ansprüche, dadurch gekennzeichnet, daß der Sicherheitsdraht (17) aus einem biegsamen Metall gebildet ist, so daß das distale Ende der Vorrichtung vorgeformt werden kann.

**Revendications**

1. Mandrin-guide comprenant:

i) un élément-âme oblong métallique flexible (12) comportant un tronçon proximal cylindrique (12a) et un tronçon effilé (12b) attenant à l'extrémité distale du tronçon proximal cylindrique;

ii) un élément enroulé en hélice (16) qui est fixé à l'élément-âme (12), entoure l'élément-âme (12) et lui est généralement coaxial et dépasse au-delà de l'extrémité distale de l'élément-âme (12), lequel élément enroulé (16) porte un bout métallique arrondi (18) à son extrémité distale libre; et

iii) un fil métallique de sûreté (17) qui est fixé au bout arrondi (18), s'étend à l'intérieur de l'élément enroulé (16) et est fixé à l'élément-âme (12);

caractérisé en ce que:

iv) l'élément-âme (12) présente un tronçon distal aplati (12c) attenant à l'extrémité distale du tronçon effilé (12b); et

v) le fil métallique de sécurité (17) est fixé à l'élément-âme (12) en un emplacement intermédiaire et espacé par rapport au tronçon proximal (12a) et au tronçon distal aplati (12c) de l'élément-âme.

2. Mandrin-guide selon la revendication 1, caractérisé en ce que le fil métallique de sécurité (17) est fixé au tronçon effilé (12b) de l'élément-âme (12).

3. Mandrin-guide selon la revendication 1 ou 2, caractérisé en ce qu'un second élément métallique enroulé en hélice (13) est fixé à l'élément-âme (12), s'étend autour du tronçon proximal (12a) et est généralement coaxial à l'élément-âme (12) et attenant au premier élément enroulé (16).

4. Mandrin-guide selon la revendication 3, caractérisé en ce qu'une composition à braser (19) lie l'une à l'autre les extrémités attenantes des premier et second éléments enroulés (16, 13).

5. Mandrin-guide selon l'une des revendications précédentes, caractérisé en ce que les spires du premier élément enroulé (16) proches de l'extrémité distale sont plus espacées les unes des autres que les spires proches de l'extrémité proximale.

6. Mandrin-guide selon l'une quelconque des revendications précédentes, caractérisé en ce que le fil métallique de sécurité (17) est en métal pliable permettant de préconformer l'extrémité distale du dispositif.

FIG.—1

FIG.—2

FIG.—3